# DEMANDE DE BREVET EUROPEEN

(11) **EP 4 702 997 A1**
(43) Date de publication de la demande: **04.03.2026**
(21) Numéro de dépôt: 24196802.3
(22) Date de dépôt: 27.08.2024
(51) Int. Cl.: A61M 5/178, G21F 5/018

(54) **BLINDAGE DE SERINGUE VERROUILLABLE**

(71) Demandeur: Trasis S.A., 4430 Ans (BE)
(72) Inventeur: MORELLE, Jean-Luc, 4000 LIEGE (BE); BAPLUE, Frédéric, 4557 FRAITURE (Tinlot) (BE)
(74) Mandataire: AWA Benelux

(57) **Abrégé**

La présente invention se rapporte à un dispositif de radioprotection (1) pour une seringue contenant une solution radioactive comprenant :
- un corps de blindage cylindrique (2) constitué d'un matériau radioprotecteur, ouvert à chacune de ses extrémités (3, 5), une extrémité en amont (5) comprenant une section de cylindre de diamètre extérieur plus petit que celui du corps de blindage cylindrique (2), ladite section de cylindre présentant des rainures (6) et possédant en son sommet deux protubérances arquées (7) aptes à épouser la forme des ailettes (22) de la seringue ; et une extrémité en aval (3) de forme conique apte à accueillir l'embout de la seringue ;
- un système rotatif de sécurisation (10) de la position de la seringue dans le corps de blindage cylindrique (2) apte à coopérer avec ladite extrémité amont (5) pour placer le système rotatif de sécurisation (10) dans une pluralité de positions définies, dont au moins une position de libération de la seringue et au moins une position de blocage de la seringue via ses ailettes (22) par rapport au corps de blindage cylindrique (2).

## Description

### Objet de l'invention

La présente invention appartient au domaine de la radioprotection, et se rapporte à un blindage de seringue. Plus spécifiquement, l'invention concerne un dispositif de maintien et de manutention de seringue blindé pour le remplissage d'une seringue avec une solution radioactive.

Le type de seringue apte à être utilisé dans le cadre de l'invention est généralement une seringue en plastique à usage unique comportant un tube muni d'un piston à une extrémité et qui est terminé à l'autre extrémité par une ouverture sur laquelle se fixe une aiguille creuse ou un petit tube. L'extrémité du tube accueillant le piston est généralement munie d'ailettes latérales qui permettent une manipulation du piston plus aisée par l'opérateur. Il s'agit par exemple des seringues d'injection standard fabriquées par BD^{®}.

### Etat de la technique

Dans le domaine de la médecine nucléaire, les préparations radiopharmaceutiques destinées à être injectées aux patients, pour l'imagerie ou le traitement de diverses maladies ou dysfonctionnements de l'organisme, contiennent des atomes radioactifs qui émettent des particules (alpha, beta) et des rayonnements ionisants (X, gamma). Ces émissions peuvent endommager les cellules et l'ADN des personnes exposées, augmentant ainsi le risque de cancer et d'autres maladies graves. Une exposition prolongée ou à forte dose à ces rayonnements peut également entraîner des effets aigus tels que des brûlures radiologiques, des maladies de radiation aiguë et des dysfonctionnements organiques.

En particulier, les professionnels de la santé responsables du remplissage de seringues avec des préparations radiopharmaceutiques pour injection au patient sont particulièrement exposés. Pour leur protection, il est donc primordial que ces seringues, pendant leur remplissage, leur transport ensuite jusqu'au patient ou site de dispensation et lors de la phase d'injection de la préparation radiopharmaceutique, soient recouvertes d'un dispositif blindé de maintien et de manutention de la seringue fabriqué à partir d'un matériau qui empêche une radioactivité excessive de s'échapper de la seringue dans l'environnement immédiat.

Les dispositifs de radioprotection de ce type actuellement disponibles sur le marché consistent en une enveloppe de forme sensiblement cylindrique, en matériau radioprotecteur (plomb, tungstène, etc.), destinée à recouvrir ou entourer essentiellement le corps cylindrique de la seringue. Cette enveloppe protectrice comporte à son extrémité avant un orifice qui permet le passage de l'aiguille d'injection ou du cône de réception de cette aiguille. Elle comporte également à son extrémité arrière un orifice qui sert à l'insertion et au retrait du corps de la seringue, ainsi qu'à l'actionnement de la tige du piston de la seringue.

Le document US 11556216 présente un dispositif de maintien de seringue blindé. Un vitrage blindé est présent dans le dispositif, de sorte que l'opérateur puisse lire le niveau de volume dans la seringue. Ce dispositif de maintien est néanmoins fait pour être posé verticalement, ce qui présente un risque de déséquilibre important et ne permet pas une connexion aisée de la seringue aux dispositifs de transfert (aiguille, check-valve, etc.). En outre, le système de sécurisation de la seringue dans ce dispositif doit se faire par manipulation manuelle du piston de la seringue. Or, faire tourner le corps de seringue à l'aide du piston uniquement (qui doit être relevé) requiert une force importante, un risque de cassure du piston de la seringue, et surtout expose l'opérateur aux radiations du contenu de la seringue par l'ouverture haute du dispositif de maintien de seringue blindé.

Le document US 7351227 B2 présente un blindage de seringue en deux parties qui coulissent l'une sur l'autre. Une fois insérée dans la partie interne du dispositif, la seringue y est fixée grâce des loquets pivotants. La manutention du corps de seringue, pour la remplir et la vider, se fait ensuite à l'aide de deux protubérances situées à l'opposé l'une de l'autre sur la partie interne du dispositif blindé. La manutention horizontale ou le long d'une table d'un paravent blindé en salle d'injection par exemple n'est dons pas aisée. En outre, il n'y a pas de mécanisme de blocage et de sécurisation du coulissement de la partie interne dans la partie externe du dispositif, les deux parties pouvant se désolidariser aisément, ce qui va à l'encontre de l'objectif visé. Un dispositif blindé en une pièce est souhaité. Dans ce même brevet US 7351227 B2, une autre variante de ce blindage présente un cylindre qui peut se fermer à l'aide de deux demi-tablettes pouvant être rapprochées manuellement l'une de l'autre, en glissant le long d'un berceau, jusqu'à encercler le piston de seringue qui peut alors être opéré manuellement. Dans cette variante, il n'y a par contre pas de sécurisation de la fermeture de ces deux demi-tablettes. Le document US6589158B2 présente également un dispositif de blindage de seringue qui présente deux membres rotatifs pouvant s'écarter l'un de l'autre pour permettre le placement de la seringue, et pouvant ensuite être ramenés l'un vers l'autre pour bloquer la seringue.

Le document WO 2023/180445A1 présente un dispositif de radioprotection destiné à équiper une seringue servant à l'injection d'un produit radioactif. Ce dispositif de radioprotection comprend une structure tubulaire de radioprotection muni d'un logement latéral pour l'accueil d'un écran en matériau radioprotecteur transparent. L'une des extrémités de l'écran comprend une structure de tenon adaptée pour coopérer avec une structure de mortaise complémentaire ménagée dans ledit logement latéral, et un organe de verrouillage escamotable est adapté pour coopérer avec une autre extrémité dudit écran en matériau radioprotecteur transparent, pour verrouiller ce dernier en position. La seringue, lorsqu'elle est placée dans le dispositif, est maintenue en place soit à l'aide d'un loquet pivotant agissant sur l'une des ailette de la seringue, soit à l'aide d'un pointeau de verrouillage dont la pointe est adaptée pour coopérer avec le corps de seringue.

Le document WO 20121/68586A1 concerne un dispositif de radioprotection destiné à équiper une seringue servant à l'injection de produit(s) radioactif(s). Ce dispositif de radioprotection comprend une enveloppe tubulaire de radioprotection qui comporte une pièce extérieure tubulaire, constituant au moins une partie de sa surface extérieure, réalisée en matériau plastique et de préférence encore en matériau élastomère dont la valeur de dureté Shore A est comprise entre 30 et 80. Dans ce dispositif, la seringue est maintenue en place par serrage d'une partie annulaire constrictive qui peut se déformer par appui manuel pour supprimer la force de frottement s'opposant à la translation de l'enveloppe tubulaire par rapport au corps de seringue, qui se retrouve ainsi libérée.

Sur le marché, on peut également se procurer des blindages de seringues en tungstène, comme par exemple :
https://www.vongahlen.com/products/radiopharmaceutical-packaging/hotlab-furniture-and-accessories/personal-protection/tunasten-syrinae-shields-(pet)).

Dans ces systèmes, la tenue de la seringue dans le blindage de seringue s'effectue à l'aide soit d'un pointeau de verrouillage, soit d'une petite vis dont la pointe est adaptée pour coopérer avec le corps de seringue, ce qui présente un risque de fuite.

Outre son rôle de radioprotection, il est important que le design du dispositif de blindage de seringue permette un positionnement sécurisé de la seringue dans le blindage et une manutention aisée de la seringue ensuite lorsqu'elle est insérée dans le blindage, tant pour des mouvements d'aspiration que d'expulsion, que pendant le transport, ce que permet avantageusement la présente invention.

### Buts de l'invention

La présente invention vise à fournir un dispositif de radioprotection pour une seringue contenant une solution radioactive.

Le dispositif de l'invention a pour but de permettre à la fois le positionnement adéquat d'une seringue dans un blindage de seringue, une sécurisation aisée ensuite de la position de la seringue dans ledit blindage et une protection maximale des opérateurs vis-à-vis des rayonnements lors du transport de la seringue ou lors des mouvements d'aspiration et/ou d'expulsion d'une solution radioactive à partir de la seringue.

### Principaux éléments caractéristiques de l'invention

La présente invention se rapporte à un dispositif de blindage d'une seringue composée d'un corps de seringue et d'un piston, permettant la manipulation sécurisée d'une solution radioactive et qui comprend un corps de blindage cylindrique dans sa majeure partie avec une ouverture à chaque extrémité, dans lequel la seringue peut être insérée, le corps de blindage comprenant en outre une fenêtre de lecture étanche aux radiations. Une des extrémités ouvertes du corps de blindage est légèrement étranglée pour accueillir l'embout de la seringue qui peut être à convenance et sans distinction par exemple soit de type luer-slip, soit de type luer-lock. L'autre extrémité du corps de blindage présente une forme adaptée à celle des ailettes de la seringue pour n'autoriser qu'un positionnement adéquat et déterminé de la seringue dans le dispositif de blindage de la seringue. A cette même extrémité, un système innovant, rotatif, blindé également, de sécurisation du positionnement de la seringue est présent. Le corps du blindage de seringue est en outre avantageusement légèrement tronqué pour permettre avantageusement un positionnement horizontal sur une table de travail. L'ensemble du blindage de la seringue de la présente invention, ainsi que la fenêtre de lecture sont avantageusement constitués de matériaux protégeant des radiations. Tant le positionnement de la seringue, que la sécurisation de sa position, que le transport de l'ensemble seringue - blindage de seringue, que l'utilisation de la seringue se font de manière entièrement protectrice pour l'opérateur.

Plus précisément, la présente invention a pour objet un dispositif de radioprotection pour une seringue contenant une solution radioactive comprenant :
- un corps de blindage cylindrique constitué d'un matériau radioprotecteur, ouvert à chacune de ses extrémités, une extrémité en amont comprenant une section de cylindre de diamètre extérieur plus petit que celui du corps de blindage cylindrique, et une extrémité en aval de forme conique apte à accueillir l'embout de la seringue ; et
- un système rotatif de sécurisation de la position de la seringue dans le corps de blindage cylindrique apte à coopérer rotativement avec ladite extrémité amont pour placer le système rotatif de sécurisation dans une pluralité de positions définies, dont au moins une position de libération de la seringue et au moins une position de blocage de la seringue via ses ailettes par rapport au corps de blindage cylindrique.

Selon des formes d'exécution préférées de l'invention, le dispositif comporte en outre au moins une des caractéristiques suivantes ou une combinaison appropriée de plusieurs d'entre elles :
- ladite section de cylindre du corps de blindage cylindrique présente des rainures et possède en son sommet deux protubérances arquées aptes à épouser la forme des ailettes de la seringue et à bloquer celles-ci dès que la seringue est introduite dans le corps de de blindage cylindrique ; et le système rotatif de sécurisation comprend un anneau inséré dans une pièce interne et solidaire de celle-ci, la pièce interne ayant une ouverture correspondant à la forme des ailettes, l'ensemble étant fixé sur l'extrémité en amont du corps de blindage cylindrique par insertion de goupilles au travers d'orifices circulaires correspondants pourvus tant dans l'anneau que dans la pièce jusqu'à pénétrer dans les rainures de l'extrémité en amont du corps de blindage cylindrique ;
- le matériau radioprotecteur du corps de blindage cylindrique et du système rotatif de sécurisation est sélectionné dans le groupe constitué du plomb et du tungstène ;
- le dispositif comprend une vitre en verre blindé disposée longitudinalement par rapport au corps de blindage cylindrique, de préférence de même épaisseur que ledit corps du blindage cylindrique, pour permettre la lecture de graduations de la seringue ;
- le vitrage de la vitre en verre blindé est constitué de verre chargé en oxyde de cérium ou de verre chargé d'oxyde de plomb ;
- le corps de blindage cylindrique présente en outre sur sa surface externe une troncature permettant de stabiliser le dispositif couché en position horizontale ;
- deux plots sont positionnés de part et d'autre de la face tronquée du corps de blindage cylindrique pour augmenter la stabilité en position horizontale du blindage de seringue ;
- le dispositif comporte deux positions définies par le système rotatif de sécurisation, une position ouverte passante pour les ailettes et permettant l'insertion ou le retrait de la seringue et une position fermée, qui place l'ouverture de la pièce perpendiculairement aux ailettes de la seringue, cette dernière se retrouvant ainsi coincée dans le blindage de seringue, par rotation du système rotatif de sécurisation d'un quart de tour ou 90 degrés dans le sens anti-horloger à partir de la position ouverte ;
- le dispositif comporte un système de butée à bille permettant le positionnement et le blocage du système rotatif de sécurisation dans l'une des positions permises ;
- le système de butée à bille comprend une bille surmontant un ressort positionné dans une encoche tubulaire présente dans l'épaisseur extérieure du corps de blindage de seringue, coopérant avec deux encoches hémisphériques présentes dans l'anneau, les rainures étant configurées pour limiter l'extension du mouvement de rotation à un intervalle [0-90 degrés] ;
- à l'intérieur de l'extrémité aval du corps de blindage cylindrique, le diamètre interne est réduit grâce à un épaulement qui empêche la seringue de ressortir du blindage de seringue par cette extrémité.

### Brève description des figures

La figure 1 montre schématiquement une vue éclatée d'un mode de réalisation du dispositif de la présente invention, exhibant les principaux éléments constitutifs et inventifs dudit dispositif.
La figure 2A représente l'anneau **12** qui fait partie du système rotatif de sécurisation de la seringue selon la présente invention.
La figure 2B représente la pièce **13** dans laquelle s'insère l'anneau **12** pour former le système de rotatif de sécurisation.
La figure 3 représente une vue du dessus et une vue du dessous d'un mode de réalisation du système rotatif de sécurisation **10** de la seringue.
La figure 4 montre une vue assemblée d'un mode de réalisation du dispositif complet selon la présente invention.
La figure 5 montre le changement de position du système rotatif de sécurisation selon la présente invention entre une position originelle ouverte (gauche), permettant l'insertion ou le retrait de la seringue et une position fermée (droite), dans laquelle la seringue est bloquée.

### Description détaillée de l'invention

Tenant compte des inconvénients précités des dispositifs de l'état de l'art, la présente invention vise à fournir un blindage de seringue qui permette de limiter l'exposition de l'opérateur aux radiations lorsque celui-ci manipule une solution radioactive à l'aide d'une seringue. La manipulation d'une solution radioactive comprend l'aspiration de la solution dans la seringue et/ou son transport dans la seringue du lieu de prélèvement vers le lieu d'injection et/ou son expulsion depuis la seringue. Un autre objet de l'invention vise à éviter tout risque d'exposition fortuite en sécurisant facilement et sans erreur possible le positionnement de la seringue dans le corps du blindage de seringue.

La présente invention est illustrée de manière détaillée dans les exemples non limitatifs à la suite.

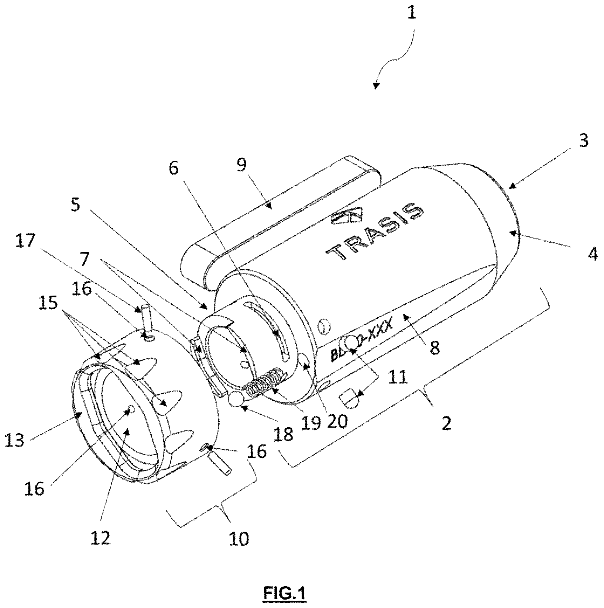

Le blindage de seringue **1** suivant un mode de réalisation de la présente invention comprend (Fig. 1) :
- un corps de blindage cylindrique **2** ouvert à chacune de ses extrémités. L'extrémité en aval **3** du corps de blindage où se situera l'embout de la seringue présente une forme conique **4,** de sorte que le diamètre extérieur de cette extrémité **3** soit inférieur à celui du corps du blindage de seringue. A l'intérieur de cette extrémité **3,** le diamètre interne est raccourci par un épaulement (non représenté) qui empêche la seringue de ressortir du blindage de seringue par cette extrémité. L'extrémité en amont **5,** qui est une section de tube de diamètre extérieur plus petit que le corps du blindage de seringue, présente trois rainures **6** et possède en son sommet deux protubérances arquées **7** qui sont aptes à épouser et bloquer les ailettes de la seringue, une fois que celle-ci est insérée dans le corps de blindage **2.** Le corps du blindage cylindrique **2** de seringue présente en outre une légère troncature **8** pour permettre de stabiliser le dispositif en position horizontale ;

- une vitre en verre blindé **9,** de préférence de même épaisseur que le corps du blindage de seringue, pour permettre la lecture des graduations de la seringue ; et
- un système rotatif blindé de sécurisation **10** de la position de la seringue dans le corps de seringue (voir ci-dessous).

Dans une forme d'exécution préférée, deux petits plots **11** sont positionnés de part et d'autre de la face tronquée du corps de blindage de seringue pour augmenter la stabilité du blindage de seringue en position horizontale.

Le corps de blindage cylindrique **2** du blindage de seringue tout comme le système rotatif de sécurisation **10** de la position de la seringue sont constitués de matériaux résistant aux rayonnements, comme le plomb ou le tungstène. Dans un mode de réalisation préféré de la présente invention, le matériau résistant aux rayonnement est le tungstène. Le corps du blindage cylindrique **2** est d'une épaisseur avantageusement comprise entre 5 mm et 15 mm.

La vitre blindée doit être suffisamment transparente pour permettre la lecture des graduations de la seringue, tout en étant étanche aux radiations. Encore suivant un mode de réalisation préféré de la présent invention, le vitrage blindé est constitué de verre chargé en oxyde de cérium ou chargé d'oxyde de plomb, d'une épaisseur identique à celle du corps de blindage cylindrique **2** dans lequel il s'insère, comprise entre 5 et 15 mm.

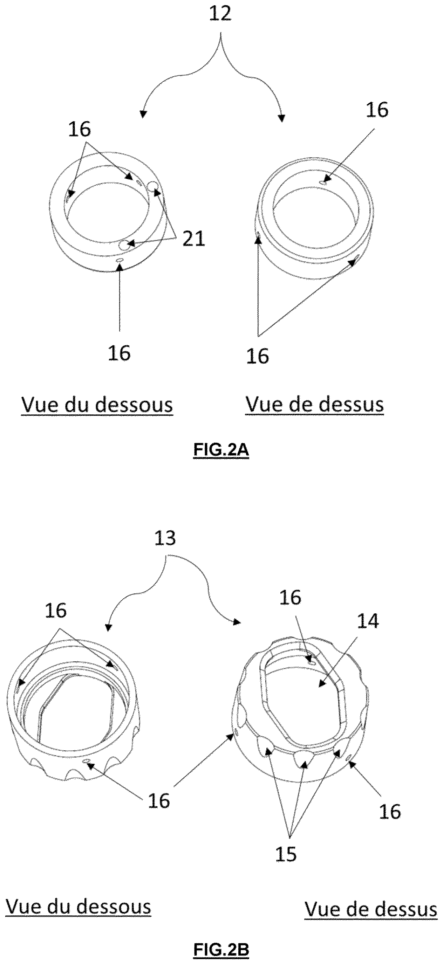

Suivant une forme d'exécution de la présente invention, le système rotatif blindé de sécurisation **10** de la seringue dans le corps de blindage de seringue est constitué d'une bague cylindrique **12** (Fig. 2A) qui s'insère dans une pièce **13** (Fig. 2B) dont l'ouverture dans une partie supérieure **14** est de forme similaire à celle des ailettes d'une seringue, de telle sorte que la seringue ne peut passer dans cette pièce que d'une seule façon. Optionnellement la partie supérieure de la pièce **13** présente de légères encoches **15** distribuées symétriquement sur toute la circonférence pour permettre une bonne prise en main. Tant l'anneau **12** que la pièce **13** sont constitués d'un matériau résistant aux radiations, tel que le plomb ou le tungstène. Dans un mode de réalisation préférée de la présente invention, le diamètre externe de la pièce **13** est le même que celui du corps de blindage cylindrique **2,** et le diamètre interne de l'anneau **12** est très légèrement supérieur au diamètre externe de l'extrémité en amont **5** du corps de blindage cylindrique **2** de manière à assurer une insertion fine de cette extrémité **5** dans l'anneau **12.**

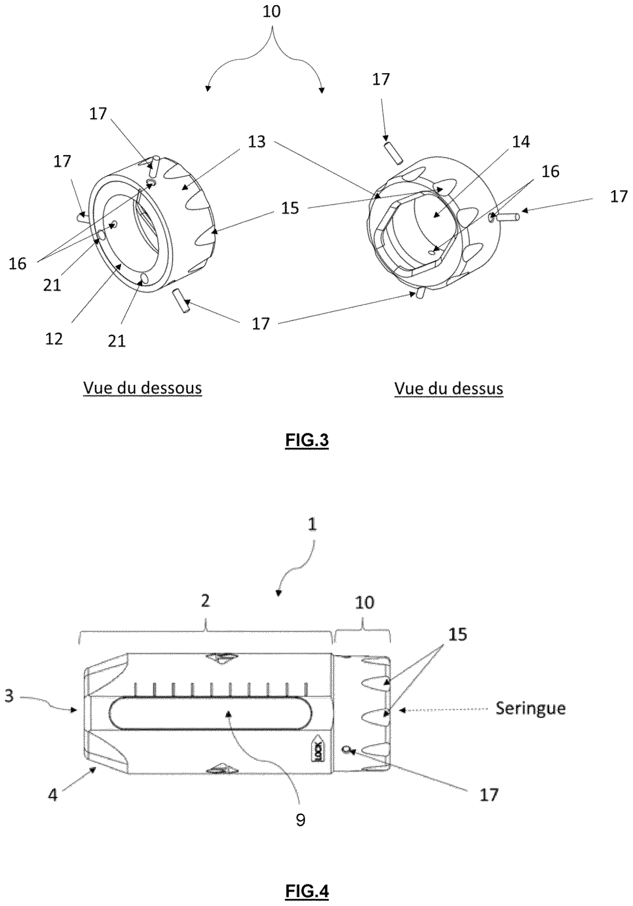

L'ensemble formé par l'insertion de l'anneau **12** dans la pièce **13,** qui se fait de manière à faire communiquer entre eux les petits orifices circulaires correspondants **16** présents dans l'anneau **12** et la pièce **13,** constitue le système rotatif blindé de sécurisation **10** de la seringue présenté à la figure 3. Cet ensemble **10** est fixé sur l'extrémité en amont **5** du corps de blindage cylindrique **2** par insertion de goupilles **17** à travers les orifices circulaires **16** jusqu'à pénétrer dans les rainures **6** de l'extrémité en amont **5** du corps de blindage cylindrique. Les goupilles **17** sont rentrées jusqu'à butée extérieure de la pièce **13.** De ce fait, le système de sécurisation **10** est indissociable du corps de blindage cylindrique **2** pour ne former qu'une entité que constitue le dispositif **1** de la présente invention comme présenté à la figure 4.

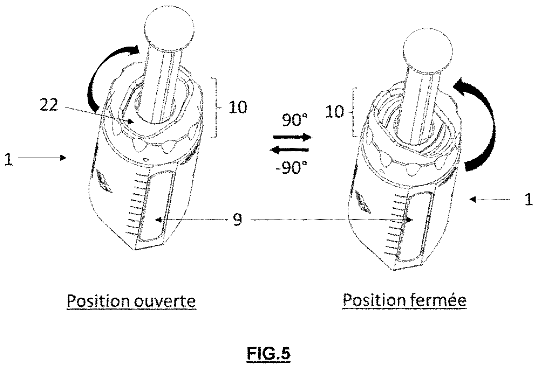

Avantageusement, une bille **18** surmontant un ressort **19** positionné dans une encoche tubulaire **20** présente dans l'épaisseur extérieure du corps de blindage de seringue **2,** ainsi que deux encoches hémisphériques **21** présentes dans l'anneau **12** en vis-à-vis, permettent la mise en place d'un système de butée à bille qui permet d'ancrer le système rotatif de sécurisation **10** dans une de deux positions permises. Grâce aux rainures **6** qui sont conçues pour limiter l'étendue possible du mouvement de rotation à un intervalle [0-90°] et à ce système de butée à bille, l'opérateur peut modifier facilement la position relative du système de rotation **10** entre une position originelle ouverte (Fig. 5 gauche), permettant l'insertion ou le retrait de la seringue, et une position fermée (Fig. 5 droite), qui place l'ouverture **14** de la pièce **13** perpendiculairement aux ailettes **22** de la seringue qui se retrouve ainsi coincée dans le blindage de seringue **1,** par rotation du système rotatif de sécurisation **10** d'un quart de tour dans le sens anti-horloger à partir de la position ouverte. Le système de butée à bille permet de verrouiller la position du système rotatif de sécurisation **10** dans une de ces deux positions, empêchant tout mouvement de rotation fortuit pendant le transport du blindage de seringue ou pendant la manutention de la seringue. Lors du transfert de la solution radioactive depuis la seringue, l'opérateur peut libérer la seringue en repositionnant le système rotatif de sécurisation **10** en position ouverte par rotation d'un quart de tour dans le sens horloger. Pendant ces manipulations, l'opérateur est protégé puisque l'opération du système rotatif de sécurisation se fait en positionnant les doigts sur la pièce **13** perpendiculairement à la seringue, et non sur une partie de la seringue elle-même.

### Liste des symboles de référence

- 1: blindage de seringue
- 2: corps de blindage cylindrique
- 3: extrémité aval
- 4: partie conique pour embout de seringue
- 5: extrémité amont
- 6: rainure circulaire (90°)
- 7: protubérance arquée
- 8: troncature
- 9: vitre en verre blindé
- 10: système rotatif de sécurisation
- 11: plot
- 12: bague cylindrique
- 13: pièce externe de système rotatif de sécurisation
- 14: partie supérieure avec ouverture adaptée aux ailettes
- 15: encoche
- 16: orifice circulaire
- 17: goupille
- 18: bille
- 19: ressort
- 20: encoche tubulaire
- 21: encoche hémisphérique
- 22: ailette de seringue

## Revendications

1. Dispositif de radioprotection **(1)** pour une seringue contenant une solution radioactive comprenant :
- un corps de blindage cylindrique **(2)** constitué d'un matériau radioprotecteur, ouvert à chacune de ses extrémités **(3, 5),** une extrémité en amont **(5)** comprenant une section de cylindre de diamètre extérieur plus petit que celui du corps de blindage cylindrique **(2),** et une extrémité en aval **(3)** de forme conique apte à accueillir l'embout de la seringue ; et
- un système rotatif de sécurisation **(10)** de la position de la seringue dans le corps de blindage cylindrique **(2)** apte à coopérer rotativement avec ladite extrémité amont **(5)** pour placer le système rotatif de sécurisation **(10)** dans une pluralité de positions définies, dont au moins une position de libération de la seringue et au moins une position de blocage de la seringue via ses ailettes **(22)** par rapport au corps de blindage cylindrique **(2).**

2. Dispositif selon la revendication 1, **caractérisé en ce que** :
- ladite section de cylindre du corps de blindage cylindrique **(2)** présente des rainures **(6)** et possède en son sommet deux protubérances arquées **(7)** aptes à épouser la forme des ailettes **(22)** de la seringue et à bloquer celles-ci dès que la seringue est introduite dans le corps de de blindage cylindrique **(2) ;**
- le système rotatif de sécurisation **(10)** comprend un anneau **(12)** inséré dans une pièce interne **(13)** et solidaire de celle-ci, la pièce interne **(13)** ayant une ouverture **(14)** correspondant à la forme des ailettes **(22),** l'ensemble **(10)** étant fixé sur l'extrémité en amont **(5)** du corps de blindage cylindrique **(2)** par insertion de goupilles **(17)** au travers d'orifices circulaires correspondants **(16)** pourvus tant dans l'anneau **(12)** que dans la pièce **(13)** jusqu'à pénétrer dans les rainures **(6)** de l'extrémité en amont **(5)** du corps de blindage cylindrique.

3. Dispositif selon la revendication 1, **caractérisé en ce que** le matériau radioprotecteur du corps de blindage cylindrique **(2)** et du système rotatif de sécurisation **(10)** est sélectionné dans le groupe constitué du plomb et du tungstène.

4. Dispositif selon l'une quelconque des revendications 1 à 3, **caractérisé en ce qu'**il comprend une vitre en verre blindé **(9)** disposée longitudinalement par rapport au corps de blindage cylindrique **(2),** de préférence de même épaisseur que ledit corps du blindage cylindrique **(2),** pour permettre la lecture de graduations de la seringue.

5. Dispositif selon la revendication 4, **caractérisé en ce que** le vitrage de la vitre en verre blindé **(9)** est constitué de verre chargé en oxyde de cérium ou de verre chargé d'oxyde de plomb.

6. Dispositif selon l'une quelconque des revendications 1 à 5, **caractérisé en ce que** le corps de blindage cylindrique **(2)** présente en outre sur sa surface externe une troncature **(8)** permettant de stabiliser le dispositif couché en position horizontale.

7. Dispositif selon la revendication 6, **caractérisé en ce que** deux plots **(11)** sont positionnés de part et d'autre de la face tronquée **(8)** du corps de blindage cylindrique **(2)** pour augmenter la stabilité en position horizontale du blindage de seringue.

8. Dispositif selon l'une quelconque des revendications 2 à 7, **caractérisé en ce qu'**il comporte deux positions définies par le système rotatif de sécurisation **(10),** une position ouverte passante pour les ailettes **(22)** et permettant l'insertion ou le retrait de la seringue et une position fermée, qui place l'ouverture **(14)** de la pièce **(13)** perpendiculairement aux ailettes **(22)** de la seringue, cette dernière se retrouvant ainsi coincée dans le blindage de seringue **(1),** par rotation du système rotatif de sécurisation **(10)** d'un quart de tour ou 90 degrés dans le sens anti-horloger à partir de la position ouverte

9. Dispositif selon l'une quelconque des revendications 2 à 8, **caractérisé en ce qu'**il comporte un système de butée à bille (18, 19, 20, 21) permettant le positionnement et le blocage du système rotatif de sécurisation **(10)** dans l'une des positions permises.

10. Dispositif selon la revendication 9, **caractérisé en ce que** le système de butée à bille comprend une bille **(18)** surmontant un ressort **(19)** positionné dans une encoche tubulaire **(20)** présente dans l'épaisseur extérieure du corps de blindage de seringue **(2),** coopérant avec deux encoches hémisphériques **(21)** présentes dans l'anneau **(12),** les rainures **(6)** étant configurées pour limiter l'extension du mouvement de rotation à un intervalle [0-90 degrés].

11. Dispositif selon l'une des revendications précédentes, **caractérisé en ce qu'**à l'intérieur de l'extrémité aval **(3)** du corps de blindage cylindrique **(2),** le diamètre interne est réduit grâce à un épaulement qui empêche la seringue de ressortir du blindage de seringue par cette extrémité.
